# EUROPEAN PATENT SPECIFICATION

(11) **EP 4 385 455 B1**
(45) Date of publication and mention of the grant of the patent: **07.05.2025**
(21) Application number: 22213439.7
(22) Date of filing: 14.12.2022
(51) Int. Cl.: A61C 13/00, A61C 9/00, A61C 19/045

(54) **METHOD OF GENERATING MANUFACTURING PARAMETERS DURING A DENTAL PROCEDURE FOR A DENTAL PROSTHESIS**
VERFAHREN ZUR ERZEUGUNG VON HERSTELLUNGSPARAMETERN WÄHREND EINES ZAHNÄRZTLICHEN VERFAHRENS FÜR EINE ZAHNPROTHESE
PROCÉDÉ DE GÉNÉRATION DE PARAMÈTRES DE FABRICATION PENDANT UNE PROCÉDURE DENTAIRE POUR UNE PROTHÈSE DENTAIRE

(30) Priority: 13.12.2022 GB 202218753
(43) Date of publication of application: 19.06.2024
(73) Proprietor: Nulty, Adam Brian, Darwen BB3 3PP (GB); Van Tonder, Quintus, Norwich NR14 8QH (GB)
(72) Inventor: Nulty, Adam Brian, Darwen BB3 3PP (GB); Van Tonder, Quintus, Norwich NR14 8QH (GB)
(74) Representative: Doherty, William

(56) References cited:
- WO-A1-2022/174165
- US-A1- 2007 207 441

## Description

The present invention relates to a method of generating manufacturing parameters during a dental procedure for a dental prosthesis, particularly but not exclusively for use in restorative dental treatments including orthodontic treatments. The invention further relates to an in-situ dental prosthesis manufacturing system which is capable of implementing the said method.

### BACKGROUND TO THE INVENTION

Computer aided design (CAD) and computer aided manufacture (CAM) in the manufacture of dental prosthesis has gained increased momentum in recent years. CAD/CAM processes offer many potential benefits over traditional methods, including increased quality of the final prosthesis, removal of the gag-inducing impressions, and the possibility of a reduction on the typical three-week lead time on the manufacture of the final prosthesis.

The existing process for CAD/CAM manufacturing of bespoke dental implants is as follows. A three-dimensional static digital impression of the mouth is taken, and the relevant data is exported. This is sent to a CAD/CAM machine off site for manufacture. The process is slow, mainly due to the need for multiple manual steps in current CAD/CAM processes including, manual image processing, manual identification of the tooth margins and the need to accurately scan and capture the surrounding and occlusive teeth.

Furthermore, CAD software requires a high-performance computing device, with the set-up thereof being expensive and generally only installed on a single computer.

Examples of methods of generating manufacturing parameters in a dental context are disclosed in WO2022/174165A1 and US2007/207441A1.

It is an object of the present invention to reduce or substantially obviate the aforementioned problems.

### SUMMARY OF THE INVENTION

According to a first aspect of the invention there is provided a method of generating manufacturing parameters during a dental procedure for a dental prosthesis. The method comprises the steps of: a) intra-orally scanning a patient to generate intra-oral data; b) extra-orally scanning the patient during a mandibular motion to generate dynamic extra-oral maxillofacial data; c) resolving the intra-oral data with the dynamic extra-oral maxillofacial data to generate a combined data model; d) generating a model representation of the dental prosthesis based on the combined data model and displaying the model representation to a user; and e) generating manufacturing parameters based on the combined data model, further comprising a step prior to step c), of identifying the patient; and a further step after step d), of exporting the intra-oral data and associating it with a dental record of the patient, and characterised in that said step is performed using facial recognition based on the dynamic extra-oral maxillofacial data and/or the static extra-oral maxillofacial data.

The present invention seeks to combine digital moulds of the tooth or teeth of interest from scans taken intra-orally with scans taken extra-orally while the mandible is in motion. By combining at least these two datasets, which may be referred to as digital moulds, allows accurate determination of the prosthesis in terms of desired bite and interferences, occlusion, contact with surrounding teeth and tooth margins, at least. Such combined data models, inform a much more accurate model with reduced user input for significantly reduced processing time and demands on skilled professionals in the field of both dentistry and image analysis and manipulation.

Preferably, during step c), more than one said combined data model may be generated. During step d), more than one said model representation of the dental prosthesis may be generated based on each of the more than one combined data models respectively. A further step after step d) and before step e) may also be provided, the step involving the selection of one representation of the dental prosthesis. During step e), manufacturing parameters may then be generated based on the combined data model of the selected one representation. In this instance, multiple options of model representations and associated combined data models are generated which may differ by tooth shape whilst also offering the correct bite. This may be presented to a user as simple on-screen prompts. By providing a selection of possible prostheses to a user, which may be the patient, dental surgeon and/or dental technician, can minimise the risk of manufacturing a prosthesis which may then be rejected by a user.

In order to help minimise the risk of manufacturing a prosthesis from the generated manufacturing parameters which may not be fit for purpose, a step after step d) and before step e) of amending the combined data model based on amendments made to the model representation or the selected one representation of the dental prosthesis displayed to the user.

Optionally, during step b), the scanning of the mandibular motion is facilitated by a contrast-enhancing medium. The contrast-enhancing medium may be a liquid or a solid deformable tablet. The contrast-enhancing medium aids the ability for algorithms to identify soft tissue and hard tissue margins for the restoration.

Beneficially, during step a), the generation of intra-oral data may include intra-orally scanning a single tooth of the patient, providing a high-quality digital mould of the tooth of interest which may be the tooth being restored.

Additionally, or alternatively, during step a), the generation of intra-oral data includes intra-orally scanning a full arch of the patient's mandibular teeth and/or maxillary teeth. Full arch scans provide data that the combined data model can use to calculate the required contact that a prosthesis may need with surrounding, or adjacent teeth.

In order to inform the manufacture of a prosthesis of the correct colouring, the intra-oral data may include data indicative of the patient's tooth colouring.

Static extra-oral maxillofacial data may also inform the combined data model by step b) further including extra-orally scanning the patient while a mandible of the patient is in a closed position, and during step c) the combined data model is based on resolving the intra-oral data with both the dynamic and static extra-oral maxillofacial data. Whilst it is acknowledged that the dynamic extra-oral data could be used to generate static extra-oral data, by extra-orally scanning the patient when their mandible is in a closed position can help inform the correct bite, interferences and/or occlusion when the patient's mandible is in a restful state, which may differ from when the mandible is in motion.

Beneficially, manufacturing parameters may be determined for many different types of prosthesis, including a veneer, a crown, an inlay, an implant, and a bridge. In other instances, the type of prosthesis may include inlays, onlays, veneers, crowns, fixed partial dentures, implant abutments, and full-mouth reconstructions. Such a versatile method can help achieve sufficient throughput for a practice to warrant investment into such CAD/CAM processes which can have significant start-up costs.

Another benefit not fully realised by current CAD/CAM systems includes the possible integration of such systems with a patient's dental records. Additionally, patient-dental surgeon discussions may also be captured using the present invention, automating the capture and storage of patient consent, further streamlining dental procedures.

Optionally, a step before step e) may be provided, of fabricating a prototype the model representation or the one selected model representation of the dental prosthesis. Such prototyping may be realised by means of additive manufacturing the model representation or the one selected representation of the dental prosthesis. By prototyping the model representation or the one selected representation, the prosthesis also has the option of being manufactured in a conventional manner.

Additionally, or alternatively, such a prototype can be used to optimise the model representation, informing amendments which may be made to the model representation and associated combined data model.

According to a second aspect of the invention there is provided an in-situ dental prosthesis manufacturing system. The system comprises: an intra-oral scanning device; an extra-oral scanning device; a computing device in communication with both the intra-oral scanning device and the extra-oral scanning device, the computing device having a display and a processor; and a manufacturing device in communication with the computing device; wherein the intra-oral scanning device is configured to generate intra-oral data; wherein the extra-oral scanning device is configured to generate dynamic extra-oral maxillofacial data; wherein the processor of the computing device is configured to resolve the intra-oral data with the dynamic extra-oral maxillofacial data to generate a combined data model; facial recognition means for performing facial recognition based on the dynamic extra-oral maxillofacial data and/or the static extra-oral maxillofacial data so as to identify the patient; wherein the display of the computing device is configured to display a model representation of the dental prosthesis based on the combined data model; wherein the processor is further configured to export the intra-oral data and associate it with a dental record of the patient, and is configured to output manufacturing parameters based on the combined data model; and wherein the manufacturing device is configured to manufacture the dental prosthesis based on the combined data model.

A system which is configured to create an accurate dental prosthesis in-situ, such that patients can walk away with their permanent restoration from only one visit to the dentist can have significant benefits on the health service. By determining manufacturing parameters on a model which combines both intra-oral and dynamic extra-oral data means that the requirement for manual processing of the image can be reduced and, in some instances, completely avoided, helping to realise the intended result of in-situ dental prosthesis manufacturing.

In an optional embodiment, the processor of the computing device is further configured to generate more than one said combined data model; wherein the display is configured to display the more than one said model representation based on the more than one combined data model respectively; and wherein the computing device is further configured to receive an input from a user for selecting one combined data model based on the selected one model representation.

Beneficially, an assisting computing device may also be provided. In this instance the assistive computing device is in communication with the computing device and is configured to display the or the selected model representation, and to receive an input from an assisting user to amend the combined data model based on the or the selected, model representation. This can act to spread the computing processing across devices. Additionally, an assistive computing device and computing device can be used in parallel to allow a dental technician to make optional amendments to the model representation while a dental surgeon and/or patient can view and possibly amend the model representation on a separate device.

Optionally, the computing device is in communication with a dental record database.

Advantageously, a prototyping device may be provided in communication with the computing device and configured to prototype the model representation or the selected model representation. The prototyping device may be a three-dimensional printer.

### BRIEF DESCRIPTION OF THE DRAWINGS

For a better understanding of the present invention, and to show more clearly how it may be carried into effect, reference will now be made by way of example only to the accompanying drawings, in which:
Figure 1 shows a pictorial representation of one embodiment of an in-situ dental prosthesis manufacturing system in accordance with a second aspect of the invention;
Figure 2 shows a pictorial representation of a first step of a method of generating manufacturing parameters during a dental procedure for a dental prosthesis in accordance with a first aspect of the invention, using the system of Figure 1, and more specifically a dental surgeon intra-orally scanning a patient and generating intra-oral data;
Figure 3 shows, in sub-Figure (a) a pictorial representation of extra-oral scanning of the patient during a mandibular motion to a closed position, generating dynamic extra-oral maxillofacial data subsequent to the step shown in Figure 2, whilst sub-Figure (b) shows a pictorial representation of extra-oral scanning of the patient during the mandibular motion to an open position, generating dynamic extra-oral maxillofacial data;
Figure 4 shows a pictorial representation of a step, subsequent to that of Figure 3, of resolving the intra-oral data with the dynamic extra-oral maxillofacial data to generate a combined data model;
Figure 5 shows a pictorial representation of a step, subsequent to that of Figure 4, of selecting a type of dental prosthesis, the types of dental prosthesis including a veneer, a crown, and inlay and an implant;
Figure 6 shows an in-situ dental prosthesis manufacturing system in accordance with a second aspect of the invention, wherein on a display of a computing device of the system, there is a pictorial representation of the process of generating more than one model representation of the dental prosthesis based on more than one combined data model;
Figure 7 shows a pictorial representation of the in-situ dental prosthesis manufacturing system of Figure 6, further comprising a prototyping device;
Figure 8 shows a pictorial representation of the in-situ dental prosthesis manufacturing system of Figure 6, further comprising a manufacturing device; and
Figure 9 shows a pictorial representation of a complete method of generating manufacturing parameters during a dental procedure for a dental prosthesis in accordance with the first aspect of the invention.

### DESCRIPTION OF PREFERRED EMBODIMENTS

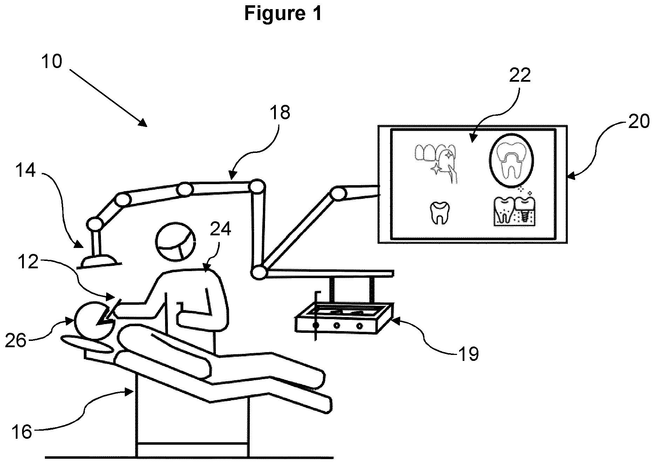

With reference to Figure 1, there is shown an in-situ dental prosthesis manufacturing system, referenced globally at 10, and which is suitable for the preparation of dental prostheses. This includes, but is not limited to, restorative prostheses such as inlays, onlays, veneers, crowns, fixed partial dentures, implant abutments, and full-mouth reconstructions, but application in orthodontics, maxillofacial treatments and the like are also within the scope of this invention.

The in-situ dental prosthesis manufacturing system 10 comprises a scanning device 12, such as a tooth sensor. In the present embodiment, this is anticipated as being a variable focal-length scanner, having replaceable sensor heads. For example, there may be a primary intra-oral camera body, having intra-oral detachable scanning elements, including a substitutable single tooth sensor head, and a full-arch sensor head. Such a scanning device 12 is used for the acquisition of intra-oral data.

The system 10 further comprises a dental light 14, here shown as an overhead scanning device mounted via an overhead support structure 18 positioned above a dental chair 16. Said overhead support structure 18 may include a support 19 for receiving the scanning device 12.

The intra-oral scanning device may also be capable as acting as an extra-oral scanning device, and which is designed to acquire extra-oral data. In this instance, the intra-oral scanning device may include an oral scanning device with detachable scanning elements. An extra-oral scanning device may be an extra-oral detachable scanning element which is compatible with the oral scanning device of the intra-oral scanning device. It will be appreciated, however, that the extra-oral scanning device 14 may be a larger device, such as a CBCT scanner, and may need to be mounted, for example, via the overhead support structure 18.

In the embodiment shown, the scanning device 12 may comprise a variable focal length scanner lens, acting as an intra-oral scanning device, which captures video, capable of translating to three-dimensional mesh information capture of the full three-dimensional facial structure and/or individual tooth shape and structure. The scanning device 12 may be configured to capture intra-oral three-dimensional anatomy and shade.

This may be provided in the form of a slim, hand-piece sized unit which may be optionally connectable directly to a computing device and/or a dental chair turbine hand-piece console for power and data transfer. A USB connection might be a typical connection means.

The camera end of the scanning device 12 may, as noted above, be replaceable in order to alter the lens type. This allows the scanning device 12 to pick up full arch and/or maxillofacial parameters, as well as providing a close-up lens for high accuracy of tooth scanning. This improves the ability to resolve the intra-oral and extra-oral data sets.

The system 10 further comprises a computing device 20 in communication with both the scanning device 12 and the extra-oral scanning device. The computing device has a display 22 and a processor.

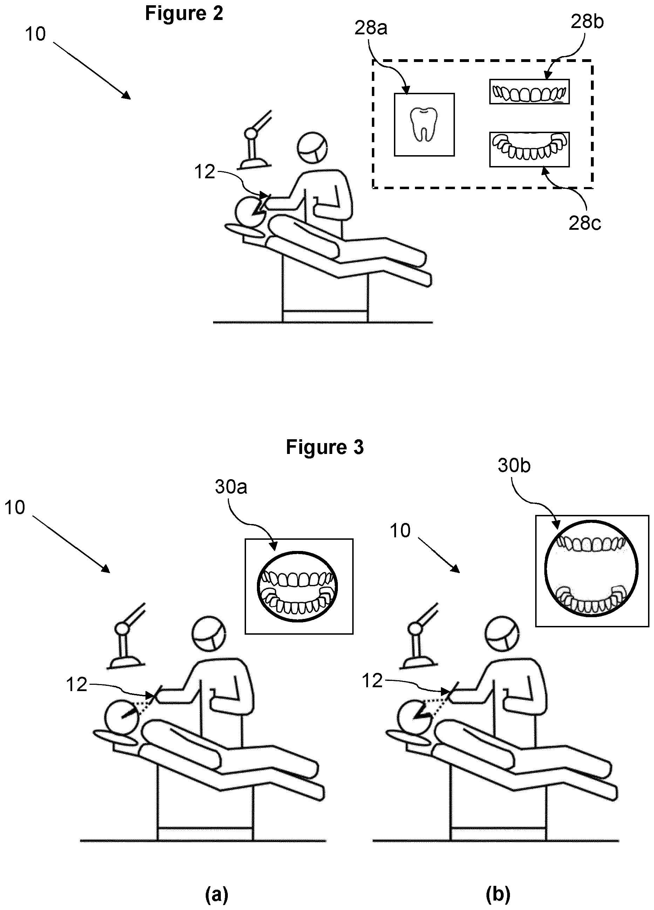

Referring now to Figure 2, a dental surgeon 24 is shown using the in-situ dental prosthesis manufacturing system 10 on a patient 26. The scanning device 12 is used to scan the patient's teeth and jaw and produce intra-oral data.

The intra-oral data in this instance comprises scan data, here illustrated as single tooth data 28a, full maxillary teeth arch data 28b, and full mandibular teeth arch data 28c. The intra-oral data can be reformatted into the form of a digital mould or otherwise into a digital reconstruction of the patient's intra-oral anatomy. In many cases, such intra-oral data is often in the form of a point-cloud, each point in the cloud having three cartesian co-ordinates identifying its location in three-dimensional space.

Whilst three types of scan data are shown, it will be appreciated that only one or combination of two or more scans or part maxillary and or mandibular teeth arch scans may be performed. Furthermore, intra-oral data comprising full or part maxillary and/or mandibular data may be generated by stitching multiple data sources together.

Although intra-oral scanning has been discussed in terms of teeth, soft tissue anatomy may also be captured to inform tooth margins and preparation margins. Intra-oral data generated from full or part maxillary and/or mandibular teeth scans can then be used to help inform contacts with adjacent teeth. Additionally, intra-oral data generated from full or part maxillary and mandibular teeth scans can be used to inform desired occlusion, bite and interference. Intra-oral data may also include data indicative of a patient's tooth colouring.

Figure 3 shows, respectively in sub-Figures (a) and (b), the mandibular motion performed during the process of extra-oral scanning. By moving the mandible, dynamic extra-oral maxillofacial data is generated, as indicated by the first and second maxillofacial scan data 30a, 30b illustrated in sub-Figures (a) and (b). Extra-oral scanning should also yield facial data, which can be achieved by performing facial recognition on the patient. This facial recognition may be performed on static extra-oral maxillofacial data, rather than on the dynamic data.

Prior to either scanning process, either intra- or extra-oral, the patient can make use of a contrast-improving composition, for example in the form of a chewable tablet or a mouthwash, which improves the definition of the margin between the teeth and the gums.

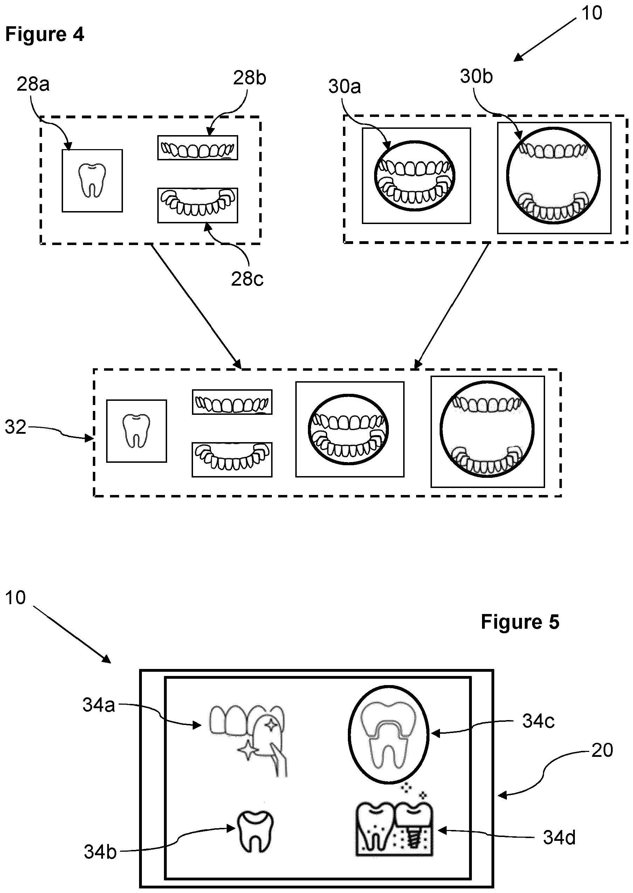

Once the intra-oral data and extra-oral maxillofacial data have been generated, they can be resolved to generate a combined data model 32. This is represented in Figure 4. From there, a model representation of a dental prosthesis can be generated. Additionally, or alternatively, it is noted that either the intra-oral data and extra-oral maxillofacial data, but most likely the intra-oral data, can be exported and associated with the standard dental records of the patient. The combined data model 32 could also be associated with the dental records of the patient.

Prior to the generation of a representation of the dental prosthesis based on the combined data model, there is a preferable step of presenting a plurality of different dental prosthesis types 34a, 34b, 34c, 34d to a user on the display 22 of the computing device 20. The user can select a type of dental prosthesis to be manufactured, in the manner shown in Figure 5 which illustrates the selection of a crown 34c. The user may be the patient, dentist, dental surgeon, or dental technician.

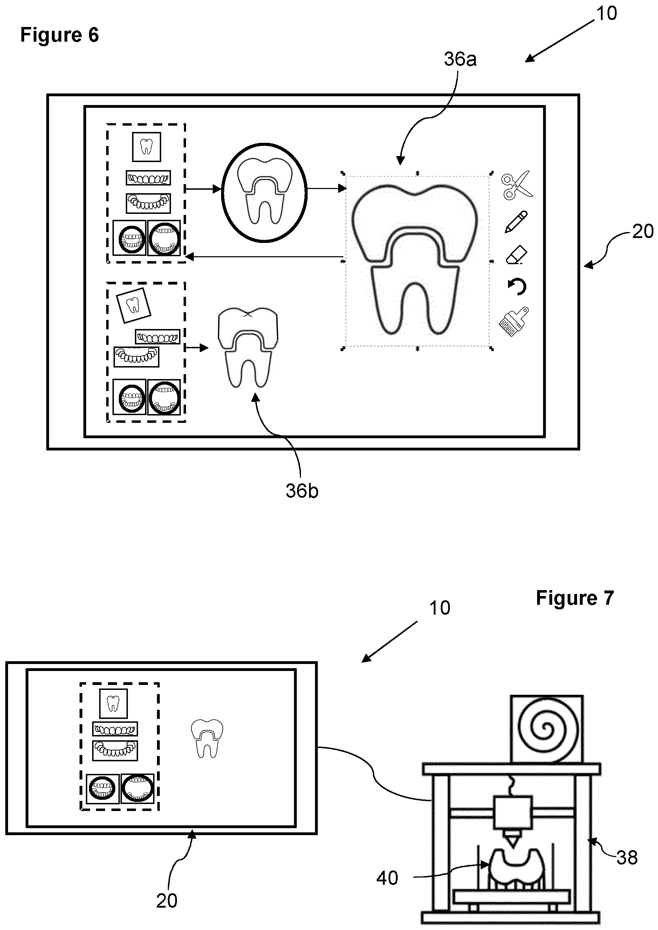

This is a powerful feature of the in-situ dental prosthesis manufacturing system 10, in that, preferably by using artificial intelligence or machine learning techniques, the representation of the dental prosthesis can be displayed to the patient, such as in the manner shown in Figure 6 via the computing device 20. Figure 6 shows two such representations of the dental prosthesis 36a, 36b based on two combined data models which are displayed to the patient, dentist, dental surgeon, or dental technician. This may display different tooth characteristics to the user, such as appearance, tooth shape, likely complexity of the dental surgical procedure, need for anaesthetic, cost, and/or longevity, which may influence the decision to be made regarding a selection of the dental prosthesis.

It will be appreciated that the selection of the desired dental prosthesis via the display 22 of the computing device 20 may be performed via a user device, such as a user smartphone. Equally, the computing device 20 could be a device located in the dental surgery, such as a tablet computer, a desktop computer, or even the dental surgeon or technician's own personal device.

The computing device 20 has a workflow thereon which operates as follows. Administrative information may be input, such as patient details, and this may be pre-populated by reception staff within the dental surgery. If the process is managed via a cloud-based server or similar networked machine, then different members of staff may be able to log in to the system to complete different tasks. Administrative information may be presented in terms of a workflow for different types of dental prosthesis, such as veneer, inlay, crown, or bridge. The type of tooth prosthesis to be designed can then also be selected, such as incisor, canine, pre-molar, or molar.

The scanning device 12 or devices can then be coupled to the computing device 20 in a wired or wireless manner, in order to import the intra-oral data and extra-oral maxillofacial data.

The processor of the computing device 20 with then be able to perform the necessary computation to resolve the combined data model.

This will be achieved typically by automatically detecting the preparation margin, thereby defining a bounding region for the combined data model. The intra-oral data can then be automatically merged with the extra-oral maxillofacial data.

In practice, this might be achieved by merging the intra-oral data with face tracking data which is a component of the extra-oral maxillofacial data, creating static composite data. This provides the system with a means of determining the physical static location of the tooth or teeth within the patient's jaw. Once this is complete, the data can be modified further using the dynamic extra-oral maxillofacial data, which provides movement data regarding the static composite data, and thus generating the full combined data model.

The combined data model will be in the form of a CAD mesh, having movement data associated therewith. An automatic artificial intelligence system can then be used to determine bite and jaw movement from the combined data model, and the automatic artificial intelligence system can modify the dental prosthesis design according to an optimised calculated bite and jaw movement. As discussed above, Figure 6 shows a representation of a display of two possible dental prosthesis options 36a, 36b for a dental prosthesis having different bite or movement characteristics, both of which being suitable for the patient following the application of the artificial intelligence calculations.

Although a selection of two representations of the dental prosthesis based on two combined data models is shown in Figure 6, it is appreciated that only one may be generated. Alternatively, more than two representations of the dental prosthesis may be generated for a greater selection. In this instance, the selection may be presented to a user by way of simple on-screen prompts.

The selected representation of the dental prosthesis 36a can be manually amended at this stage in the manner shown in Figure 6. These amendments are then reflected in the associated combined data model.

At this point, suitable manufacturing parameters can be output based on the combined data model. The computing device 20 can automatically export the manufacturing parameters, or can communicate directly with a CAM machine.

One powerful option would be to allow the computing device 20 to directly interface with a rapid prototyping machine, as shown in Figure 7. Typically, this would be an additive manufacturing apparatus 38, such as a 3D printer. This allows for in situ production of a temporary prosthesis, which can be created and installed almost immediately, without any manufacturing lead times. Such prototyping may even be used to inform manual amendments which can be made via the computing device 20.

It is expected that the workflow here would involve 3D printing of the prototype prosthesis 40, followed by a washing process and a resin cure process, to allow for prompt integration of the prototype prosthesis 40 into the patient.

This might be in the innovative form of a new form of 3D printer, in which there are two chambers: a first printing chamber, and a second wash-and-cure chamber. Once printed in the first printing chamber, the prototype prosthesis 40 is transferred via an actuator into the second wash-and-cure chamber. This is the opposite process to standard 3D printers, where the resin is allowed to fast-flow back into the curing layer to provide for improved speed. The advantage here is that the dental surgeon or technician need never come into contact with wet resin, thereby avoiding contamination.

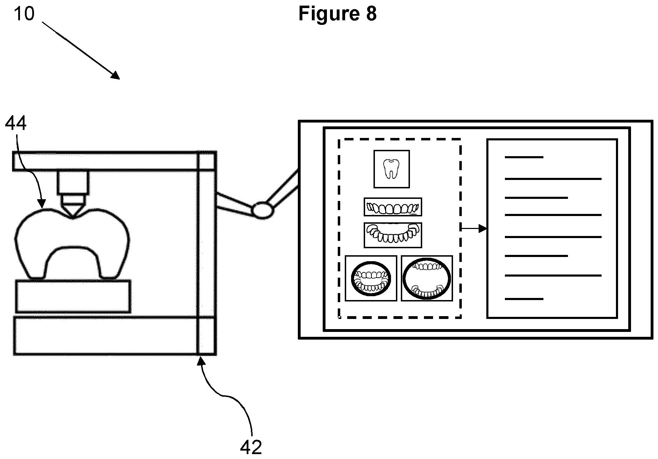

All while this process is underway, the manufacturing parameters can also be exported, as shown in Figure 8, to a standard CAM machine 42, which may be offsite, to allow for precision milling and manufacture of the final dental prosthesis 44. The patient can return at some point in the future to have their temporary prototype prosthesis 40 replaced with the more hard-wearing final dental prosthesis 44. However, the rapid manufacturing aspect of the present invention provides for a workflow in which there is no lag time where the patient has no prosthesis.

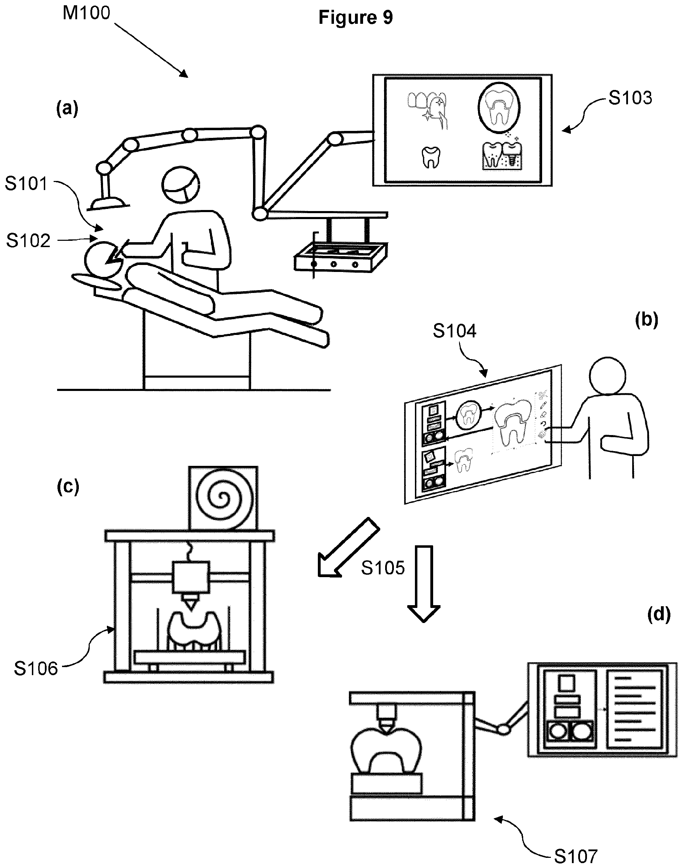

Figure 9 shows a summary of the methodology used in the present invention. There is a method M100 of generating manufacturing parameters during a dental procedure for a dental prosthesis, the method comprising the steps of: a) intra-orally scanning, at step S101, a patient to generate intra-oral data; b) extra-orally scanning, at step S102, the patient during a mandibular motion to generate dynamic extra-oral maxillofacial data; c) resolving the intra-oral data, at step S103, with the dynamic extra-oral maxillofacial data to generate a combined data model; d) generating, at step S104, a model representation of the dental prosthesis based on the combined data model and displaying the model representation to a user; and e) generating manufacturing parameters, at step S105, based on the combined data model. The manufacturing parameters can be used in situ for rapid prototyping in the dental surgery, at step S106, and meanwhile, the manufacturing parameters can be used to prepare a more detailed bespoke dental prosthesis, at step S107, for subsequent implantation.

It will be apparent that, whilst the term dental prosthesis is used primarily in relation to tooth implants or similar, that orthodontic inserts, such as alignment tools or braces, could equally be designed in situ using the system described above.

Furthermore, whilst the control of the workflow from a device-level perspective has been illustrated as being via a single computing device, it will be appreciated that there could be non-centralised data engagement across several devices

It is therefore possible to provide a method of generating manufacturing parameters during a dental procedure for a dental prosthesis such that a patient can receive a permanent dental prosthesis the same day or during the same procedure. Additionally, it is also possible to provide an in-situ dental prosthesis manufacturing system.

The words 'comprises/comprising' and the words 'having/including' when used herein with reference to the present invention are used to specify the presence of stated features, integers, steps, or components, but do not preclude the presence or addition of one or more other features, integers, steps, components, or groups thereof.

The embodiments described above are provided by way of example only, and various changes and modifications will be apparent to persons skilled in the art without departing from the scope of the present invention as defined by the appended claims.

## Claims

1. A method (M100) of generating manufacturing parameters during a dental procedure for a dental prosthesis, the method comprising the steps of:
a) intra-orally scanning (S101) a patient to generate intra-oral data;
b) extra-orally scanning (S102) the patient during a mandibular motion to generate dynamic extra-oral maxillofacial data;
c) resolving (S103) the intra-oral data with the dynamic extra-oral maxillofacial data to generate a combined data model;
d) generating (S104) a model representation of the dental prosthesis based on the combined data model and displaying the model representation to a user; and
e) generating (S105) said manufacturing parameters based on the combined data model, further comprising a step prior to step c), of identifying the patient; and a further step after step d), of exporting the intra-oral data and associating it with a dental record of the patient, and **characterised in that** said step of identifying the patient is performed using facial recognition based on the dynamic extra-oral maxillofacial data and/or static extra-oral maxillofacial data.

2. A method (M100) as claimed in claim 1, wherein during step c), more than one said combined data model is generated; wherein during step d), more than one said model representation of the dental prosthesis is generated based on each of the more than one combined data models respectively; further comprising a step after step d) and before step e), of selecting one representation of the dental prosthesis; and wherein during step e), manufacturing parameters are generated based on the combined data model of the selected one representation.

3. A method (M100) as claimed in claim 1 or claim 2, further comprising a step after step d) and before step e), of amending the combined data model based on amendments made to the model representation or the selected one representation of the dental prosthesis displayed to the user.

4. A method (M100) as claimed in any one of the preceding claims, wherein during step b), the scanning of the mandibular motion is facilitated by a contrast-enhancing medium.

5. A method (M100) as claimed in any one of the preceding claims, wherein during step a), the generation of intra-oral data includes intra-orally scanning a single tooth of the patient.

6. A method (M100) as claimed in any one of the preceding claims, wherein during step a), the generation of intra-oral data includes intra-orally scanning a full arch of the patient's mandibular teeth and/or maxillary teeth.

7. A method (M100) as claimed in any one of the preceding claims, wherein step b), further includes extra-orally scanning the patient while a mandible of the patient is in a closed position to generate static extra-oral maxillofacial data; and during step c) the combined data model is based on resolving the intra-oral data with both the dynamic and static extra-oral maxillofacial data.

8. A method (M100) as claimed in any one of the preceding claims, further comprising a step before step e), of fabricating a prototype of the model representation or the one selected model representation of the dental prosthesis.

9. An in-situ dental prosthesis manufacturing system (10), the system comprising:
an intra-oral scanning device;
an extra-oral scanning device;
a computing device (20) in communication with both the intra-oral scanning device and the extra-oral scanning device, the computing device (20) having a display (22) and a processor; and
a manufacturing device (42) in communication with the computing device;
wherein the intra-oral scanning device is configured to generate intra-oral data;
wherein the extra-oral scanning device is configured to generate dynamic extra-oral maxillofacial data;
wherein the processor of the computing device is configured to resolve the intra-oral data with the dynamic extra-oral maxillofacial data to generate a combined data model;
facial recognition means for performing facial recognition based on the dynamic extra-oral maxillofacial data and/or static extra-oral maxillofacial data so as to identify the patient;
wherein the display (22) of the computing device (20) is configured to display a model representation of a dental prosthesis based on the combined data model;
wherein the processor is further configured to export the intra-oral data and associate it with a dental record of the patient, and is configured to output manufacturing parameters for the dental prosthesis based on the combined data model:
and
wherein the manufacturing device (42) is configured to manufacture the dental prosthesis based on the combined data model.

10. An in-situ dental prosthesis manufacturing system (10) as claimed in claim 9, wherein the processor of the computing device is further configured to generate more than one said combined data model; wherein the display is configured to display the more than one said model representation based on the more than one combined data model respectively; and wherein the computing device is further configured to receive an input from a user for selecting one combined data model based on the selected one model representation.

## Patentansprüche

1. Verfahren (M100) zur Erzeugung von Herstellungsparametern während eines zahnärztlichen Eingriffs für eine Zahnprothese, wobei das Verfahren die Schritte umfasst:
a) intraorales Scannen (S101) eines Patienten, um intraorale Daten zu erzeugen;
b) extraorales Scannen (S102) des Patienten während einer Unterkieferbewegung, um dynamische extraorale maxillofaziale Daten zu erzeugen;
c) Auflösen (S103) der intraoralen Daten mit den dynamischen extraoralen maxillofazialen Daten, um ein kombiniertes Datenmodell zu erzeugen;
d) Erzeugen (S104) einer Modelldarstellung der Zahnprothese auf der Basis des kombinierten Datenmodells und Anzeigen der Modelldarstellung für einen Benutzer; und
e) Erzeugen (S105) der Herstellungsparameter auf der Basis des kombinierten Datenmodells,
weiterhin umfassend einen Schritt vor Schritt c) eines Identifizierens des Patienten; und einen weiteren Schritt nach Schritt d) eines Exportierens der intraoralen Daten und Assoziieren dieser mit Zahnunterlagen des Patienten, und **dadurch gekennzeichnet, dass** der Schritt des Identifizierens des Patienten unter Verwendung von Gesichtserkennung auf der Basis der dynamischen extraoralen maxillofazialen Daten und/oder von statischen extraoralen maxillofazialen Daten durchgeführt wird.

2. Verfahren (M100) nach Anspruch 1, wobei während Schritt c) mehr als eines des kombinierten Datenmodells erzeugt wird; wobei während Schritt d) mehr als eine der Modelldarstellung der Zahnprothese jeweils auf der Basis jedes des mehr als einen kombinierten Datenmodells erzeugt wird; weiterhin umfassend einen Schritt nach Schritt d) und vor Schritt e) eines Auswählens einer Darstellung der Zahnprothese; und wobei während Schritt e) Herstellungsparameter auf der Basis des kombinierten Datenmodells der ausgewählten einen Darstellung erzeugt werden.

3. Verfahren (M100) nach Anspruch 1 oder Anspruch 2, weiterhin umfassend einen Schritt nach Schritt d) und vor Schritt e) eines Änderns des kombinierten Datenmodells auf der Basis von Änderungen, die an der Modelldarstellung oder der ausgewählten einen Darstellung der Zahnprothese, die für den Benutzer angezeigt wird, vorgenommen werden.

4. Verfahren (M100) nach einem der vorhergehenden Ansprüche, wobei während Schritt b) das Scannen der Unterkieferbewegung durch ein Kontrastverstärkungsmittel erleichtert wird.

5. Verfahren (M100) nach einem der vorhergehenden Ansprüche, wobei während Schritt a) die Erzeugung von intraoralen Daten ein intraorales Scannen eines einzigen Zahns des Patienten beinhaltet.

6. Verfahren (M100) nach einem der vorhergehenden Ansprüche, wobei während Schritt a) die Erzeugung von intraoralen Daten ein intraorales Scannen eines vollständigen Bodens der Unterkieferzähne und/oder der Oberkieferzähne des Patienten beinhaltet.

7. Verfahren (M100) nach einem der vorhergehenden Ansprüche, wobei Schritt b) weiterhin ein extraorales Scannen des Patienten beinhaltet, während ein Unterkiefer des Patienten in einer geschlossenen Position ist, um statische extraorale maxillofaziale Daten zu erzeugen; und während Schritt c) das kombinierte Datenmodell auf einem Auflösen der intraoralen Daten mit sowohl den dynamischen als auch den statischen extraoralen maxillofazialen Daten basiert.

8. Verfahren (M100) nach einem der vorhergehenden Ansprüche, weiterhin umfassend einen Schritt vor Schritt e) eines Fertigens eines Prototyps der Modelldarstellung oder der einen ausgewählten Modelldarstellung der Zahnprothese.

9. In-situ-Zahnprothesenherstellungssystem (10), wobei das System umfasst:
eine intraorale Scanvorrichtung;
eine extraorale Scanvorrichtung;
eine Rechenvorrichtung (20) in Kommunikation mit sowohl der intraoralen Scanvorrichtung als auch der extraoralen Scanvorrichtung, wobei die Rechenvorrichtung (20) eine Anzeige (22) und einen Prozessor aufweist; und
eine Herstellungsvorrichtung (42) in Kommunikation mit der Rechenvorrichtung;
wobei die intraorale Scanvorrichtung dazu konfiguriert ist, intraorale Daten zu erzeugen;
wobei die extraorale Scanvorrichtung dazu konfiguriert ist, dynamischen extraorale maxillofaziale Daten zu erzeugen;
wobei der Prozessor der Rechenvorrichtung dazu konfiguriert ist, die intraoralen Daten mit den dynamischen extraoralen maxillofazialen Daten aufzulösen, um ein kombiniertes Datenmodell zu erzeugen;
Gesichtserkennungsmittel zum Durchführen einer Gesichtserkennung auf der Basis der dynamischen extraoralen maxillofazialen Daten und/oder von statischen extraoralen maxillofazialen Daten, um den Patienten zu identifizieren;
wobei die Anzeige (22) der Rechenvorrichtung (20) dazu konfiguriert ist, eine Modelldarstellung einer Zahnprothese auf der Basis des kombinierten Datenmodells anzuzeigen;
wobei der Prozessor weiterhin dazu konfiguriert ist, die intraoralen Daten zu exportieren und diese mit Zahnunterlagen des Patienten zu assoziieren, und dazu konfiguriert ist, Herstellungsparameter für die Zahnprothese auf der Basis des kombinierten Datenmodells auszugeben; und
wobei die Herstellungsvorrichtung (42) dazu konfiguriert ist, die Zahnprothese auf der Basis des kombinierten Datenmodells herzustellen.

10. In-situ-Zahnprothesenherstellungssystem (10) nach Anspruch 9, wobei der Prozessor der Rechenvorrichtung weiterhin dazu konfiguriert ist, mehr als eines des kombinierten Datenmodells zu erzeugen; wobei die Anzeige dazu konfiguriert ist, die mehr als eine der Modelldarstellung jeweils auf der Basis des mehr als einen kombinierten Datenmodells anzuzeigen; und wobei die Rechenvorrichtung weiterhin dazu konfiguriert ist, eine Eingabe von einem Benutzer zum Auswählen eines kombinierten Datenmodells auf der Basis der ausgewählten einen Modelldarstellung zu empfangen.

## Revendications

1. Un procédé (M100) de génération de paramètres de fabrication pendant une procédure dentaire pour une prothèse dentaire, le procédé comprenant les étapes consistant à :
a) numériser intra-oralement (SIOI) un patient pour générer des données intra-orales ;
b) numériser extra-oralement (S102) le patient pendant un mouvement mandibulaire pour générer des données maxillo-faciales extra-orales dynamiques ;
c) résoudre (S103) les données intra-orales avec les données maxillo-faciales extra-orales dynamiques pour générer un modèle de données combiné ;
d) générer (S104) une représentation de modèle de la prothèse dentaire sur la base du modèle de données combiné et afficher la représentation de modèle à un utilisateur ; et
e) générer (5105) lesdits paramètres de fabrication sur la base du modèle de données combiné, comprenant en outre une étape préalable à l'étape c), d'identification du patient ; et une étape supplémentaire après l'étape d), d'exportation des données intra-orales et de leur association à un dossier dentaire du patient, et **caractérisé en ce que** ladite étape d'identification du patient est réalisée à l'aide d'une reconnaissance faciale sur la base des données maxillo-faciales extra-orales dynamiques et/ou des données maxillo-faciales extra-orales statiques.

2. Un procédé (M100) selon la revendication 1, dans lequel, au cours de l'étape c), plusieurs modèles de données combinés sont générés ; dans lequel, au cours de l'étape d), plusieurs représentations de modèle de la prothèse dentaire sont générées sur la base de chacun de plusieurs modèles de données combinés respectivement ; comprenant en outre une étape après l'étape d) et avant l'étape e), consistant à sélectionner une représentation de la prothèse dentaire ; et dans lequel, au cours de l'étape e), des paramètres de fabrication sont générés sur la base du modèle de données combiné de la représentation sélectionnée.

3. Un procédé (M100) selon la revendication 1 ou la revendication 2, comprenant en outre une étape après l'étape d) et avant l'étape e), consistant à modifier le modèle de données combiné sur la base des modifications apportées à la représentation du modèle ou à la représentation sélectionnée de la prothèse dentaire affichée à l'utilisateur.

4. Un procédé (M100) selon l'une quelconque des revendications précédentes, dans lequel, au cours de l'étape b), la numérisation du mouvement mandibulaire est facilitée par un milieu améliorant le contraste.

5. Un procédé (M100) selon l'une quelconque des revendications précédentes, dans lequel, au cours de l'étape a), la génération de données intra-orales comprend la numérisation intra-orale d'une seule dent du patient.

6. Un procédé (M100) selon l'une quelconque des revendications précédentes, dans lequel, au cours de l'étape a), la génération de données intra-orales comprend la numérisation intra-orale d'une arcade complète des dents mandibulaires et/ou des dents maxillaires du patient.

7. Un procédé (M100) selon l'une quelconque des revendications précédentes, dans lequel l'étape b) comprend en outre la numérisation extra-orale du patient tandis qu'une mandibule du patient est dans une position fermée pour générer des données maxillo-faciales extra-orales statiques ; et pendant l'étape c), le modèle de données combiné est basé sur la résolution des données intra-orales avec les données maxillo-faciales extra-orales dynamiques et statiques.

8. Un procédé (M100) selon l'une quelconque des revendications précédentes, comprenant en outre une étape avant l'étape e), consistant à fabriquer un prototype de la représentation de modèle ou de la représentation de modèle sélectionnée de la prothèse dentaire.

9. Un système de fabrication de prothèses dentaires in situ (10), le système comprenant :
un dispositif de numérisation intra-orale ;
un dispositif de numérisation extra-orale ;
un dispositif informatique (20) en communication avec le dispositif de numérisation intra-orale et le dispositif de numérisation extra-orale, le dispositif informatique (20) ayant un écran (22) et un processeur ; et un dispositif de fabrication (42) en communication avec le dispositif informatique ; le dispositif de numérisation intra-orale étant configuré pour générer des données intra-orales ; le dispositif de numérisation extra-orale étant configuré pour générer des données maxillo-faciales extra-orales dynamiques ;
dans lequel le processeur du dispositif informatique est configuré pour résoudre les données intra-orales avec les données maxillo-faciales extra-orales dynamiques pour générer un modèle de données combiné ; des moyens de reconnaissance faciale pour effectuer une reconnaissance faciale sur la base des données maxillo-faciales extra-orales dynamiques et/ou des données maxillo-faciales extra-orales statiques de manière à identifier le patient; dans lequel l'affichage (22) du dispositif informatique (20) est configuré pour afficher une représentation de modèle d'une prothèse dentaire sur la base du modèle de données combiné ;
dans lequel le processeur est en outre configuré pour exporter les données intra-orales et les associer à un dossier dentaire du patient, et est configuré pour générer des paramètres de fabrication pour la prothèse dentaire sur la base du modèle de données combinées :et dans lequel le dispositif de fabrication (42) est configuré pour fabriquer la prothèse dentaire sur la base du modèle de données combiné.

10. Un système de fabrication de prothèse dentaire in situ (10) selon la revendication 9, dans lequel le processeur du dispositif informatique est en outre configuré pour générer plus d'un modèle de données combiné ; dans lequel l'affichage est configuré pour afficher le plus d'une représentation de modèle sur la base de plus d'un modèle de données combiné respectivement ; et dans lequel le dispositif informatique est en outre configuré pour recevoir une entrée d'un utilisateur pour sélectionner un modèle de données combiné sur la base de la représentation de modèle sélectionnée.
